(19) Europäisches Patentamt
European Patent Office
Office européen des brevets

(11) EP 0 600 060 B1

(12) # FASCICULE DE BREVET EUROPEEN

(45) Date de publication et mention de la délivrance du brevet: **23.10.1996 Bulletin 1996/43**

(21) Numéro de dépôt: **93912838.5**

(22) Date de dépôt: **01.06.1993**

(51) Int. Cl.$^6$: **A61K 7/32**, C11D 3/36, C11D 3/50, C11D 3/20, A61K 7/46

(86) Numéro de dépôt international: **PCT/EP93/01366**

(87) Numéro de publication internationale: **WO 93/25185 (23.12.1993 Gazette 1993/30)**

(54) **COMPOSITION PARFUMEE**
PARFÜMIERTES MITTEL
PERFUMED COMPOSITION

(84) Etats contractants désignés:
**CH DE ES FR GB IT LI**

(30) Priorité: **16.06.1992 CH 1889/92**

(43) Date de publication de la demande:
**08.06.1994 Bulletin 1994/23**

(73) Titulaires:
- **FIRMENICH SA**
  **CH-1211 Genève 8 (CH)**
- **Holzner, Günter Wolfgang**
  **CH-1212 Grand-Lancy (CH)**
- **BUCHLI, Franz**
  **CH-1226 Thonex (CH)**

(72) Inventeurs:
- **HOLZNER, Günter**
  **CH-1212 Grand-Lancy (CH)**
- **BUCHLI, Franz**
  **CH-1226 Thônex (CH)**

(74) Mandataire: **Salvadori, Giuseppe, Dr. et al**
**c/o Firmenich S.A.**
**Case Postale 239**
**1211 Genève 8 (CH)**

(56) Documents cités:
**EP-A- 0 347 306**
**EP-A- 0 433 132**
**EP-A- 0 451 889**
**GB-A- 2 013 609**
**US-A- 4 174 296**

- **DATABASE WPI Week 8327, Derwent Publications Ltd., London, GB; AN 83-702271 & JP,A,58 088 308 (OTOMO) 26 Mai 1983**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.13.8/3.4

## Description

### Domaine technique et art antérieur

La présente invention a trait au domaine de la parfumerie et de la cosmétique et, plus particulièrement, à celui des désodorisants et antiperspirants corporels.

La fonction principale d'un désodorisant ou antiperspirant corporel est d'empêcher les mauvaises odeurs corporelles dues à la décomposition bactérienne et à l'oxydation des substances organiques qui composent la sueur. Conventionnellement, les désodorisants contiennent des substances capables de masquer ces odeurs corporelles et d'inhiber la prolifération des bactéries responsables de la dégradation de la sueur, tandis que les antiperspirants comprennent des composants ayant une action de réduction de la transpiration, tout en exerçant une activité bactéricide.

Dans un cas, comme dans l'autre, on observe actuellement une tendance du consommateur à préférer des articles désodorisants basés sur des principes actifs naturels ou nature identiques, c'est-à-dire, à base de substances synthétisées mais de structure identique aux composés d'occurrence naturelle. Pour répondre à cette attente, on cherche à éliminer dans les articles désodorisants et antiperspirants corporels les substances bactéricides, bactériostatiques et antiperspirantes conventionnelles, souvent allergéniques, et à les remplacer par des principes actifs naturels. C'est ainsi que l'on a assisté dans les dernières années au développement de produits dont l'action bactéricide était due essentiellement à la présence de compositions parfumantes contenant des ingrédients parfumants soigneusement sélectionnés, de façon à garantir une action désodorisante adéquate, sans avoir recours à des substances bactéricides conventionnelles [voir, par exemple, G.W. Holzner dans Aerosol Report 25, 354 (1986); voir également la demande de brevet publiée EP 451 889 qui contient une analyse fort complète des connaissances de l'art dans le domaine de l'activité bactéricide des ingrédients parfumants d'origine naturelle ou synthétique].

Un autre exemple particulier d'une composition représentative de cette tendance est celui des compositions décrites dans la demande de brevet européenne n° 433 132, qui comprennent des huiles essentielles ayant une activité antibactérienne et/ou antifongique dispersées dans une phase aqueuse stabilisée à l'aide d'une phase lipidique comprenant des lipides amphiphiles anioniques ou non-ioniques.

D'autre part, un désodorisant corporel de choix doit présenter un spectre d'activité bactéricide aussi large que possible, c'est-à-dire, il doit empêcher, aussi efficacement que possible, la croissance de tous les organismes microbiens présents sur la peau. Or, on sait que les produits désodorisants à base de substances parfumantes n'ont pas une efficacité égale vis-à-vis de toutes ces variétés d'organismes.

Le but de la présente invention est d'apporter une contribution originale à ce problème.

### Exposé de l'invention

L'invention concerne une composition parfumée caractérisée en ce qu'elle contient un phospholipide cationique, une base parfumante à action antimicrobienne ("BPAA") et un alcool gras ayant de 10 à 22 atomes de carbone.

Nous avons en effet découvert que la présence simultanée de ces trois ingrédients dans la composition parfumée permettait d'inhiber l'action d'une grande variété de microorganismes et conférait à ladite composition un pouvoir désodorisant remarquable qui la rendait particulièrement adaptée pour des applications dans des articles désodorisants ou antiperspirants, comme il ressort des exemples présentés plus loin. Il a été constaté de façon surprenante que la présence de la "BPAA" et de l'alcool gras renforçait, de façon synergique, l'action des phospholipides cationiques et permettait d'obtenir des compositions parfumées améliorées par rapport à celles de l'art antérieur.

Par ailleurs, nous avons également constaté que les compositions selon l'invention pouvaient avoir un effet antiperspirant sensible, même sans recourir à l'utilisation des sels d'aluminium ou d'aluminium et zirconium, couramment employés en tant que substances antiperspirantes actives.

Les phospholipides cationiques sont des substances nature identiques que l'on utilise couramment en cosmétique en tant qu'émulsifiants ou émollients. Cependant, à notre connaissance, il n'existe aucun rapport dans la littérature ayant trait à l'utilisation de ces substances dans des désodorisants ou antiperspirants corporels. Or, nous avons maintenant découvert que les compositions parfumées selon une forme d'exécution préférée de l'invention, caractérisées en ce qu'elles présentent une activité antimicrobienne d'au moins 80%, telle que mesurée par la méthode du spray direct décrite ci-après, inhibent efficacement les mauvaises odeurs de transpiration pendant au moins douze heures lorsqu'appliquées sur la peau et évitent l'utilisation de substances telles que l'hexachlorophène, le dichlorophénol, le trichlorosalicylanilide, le tribromosalicylanilide (TBS), le tétrachlorosalicylanilide (TCSA), le trichlorocarbanilide (TCC) ou l'Irgasan ® DP-300 (Ciba-Geigy), jusqu'ici couramment utilisées en tant qu'agents bactéricides ou bactériostatiques.

Les compositions de l'invention présentent des avantages certains sur les compositions désodorisantes et antiperspirantes qui font usage des principes actifs conventionnels. En effet, les phospholipides cationiques sont des substances biodégradables non toxiques, qui ne manifestent pas d'effets irritants sur la peau mais, au contraire, ont un effet adoucissant sur celle-ci.

Selon une forme préférée d'exécution de l'invention, on peut utiliser des phospholipides d'origine synthétique, notamment ceux qui sont nature identiques. Nous avons observé que de meilleurs résultats étaient obtenus lorsque l'on utilisait des phospholipides de formule

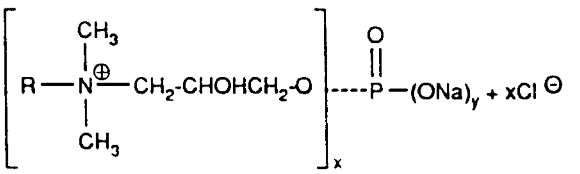

(I)

dans laquelle R est un radical linoléamidopropyle ou cocamidopropyle et $x + y = 3$ .

On trouve de tels phospholipides sur le marché, notamment sous les désignations commerciales de Phospholipid PTC et Phospholipid EFA (Mona Industries Inc., USA).

Ces phospholipides peuvent être ajoutés directement aux formulations classiques de bases désodorisantes ou antiperspirantes couramment utilisées. Des exemples de telles préparations sont présentés plus loin. De préférence, les phospholipides seront ajoutés dans des concentrations comprises entre 0,05 et 5% en poids, par rapport au poids de la composition parfumée dans laquelle ils sont incorporés.

En tant qu'alcool gras présent dans les compositions parfumées selon l'invention, on utilisera, de préférence, un alcool gras ayant de 14 à 18 atomes de carbone. Les alcools cétylique et myristique se sont révélés particulièrement avantageux pour cette application.

Selon un mode d'exécution préférentiel, l'alcool gras est ajouté dans une concentration comprise entre 0,02 et 2% en poids, par rapport au poids de la composition parfumée.

La base parfumante à action antimicrobienne, ou "BPAA", d'autre part, sera ajoutée de préférence dans une concentration comprise entre 0,1 et 5% en poids, par rapport au poids de la composition.

Selon une forme d'exécution préférentielle, la composition parfumée selon l'invention contient environ 0,05 à 2% en poids de phospholipide, environ 0,5 à 5% en poids de "BPAA" et environ 0,05 à 2% en poids d'alcool gras, par rapport au poids de composition, et présente une activité antimicrobienne d'au moins 90%, telle que mesurée par la méthode du spray direct.

Par "base parfumante à action antimicrobienne" ("BPAA") on entend ici une base parfumante constituée par un mélange de substances parfumantes, tant à l'état isolé qu'en solution ou suspension, dans leurs diluants, dissolvants ou coingrédients habituels, ces substances pouvant être choisies dans des classes chimiques variées, comprenant par exemple des esters, des éthers, des alcools, des aldéhydes, des cétones, des acétals, des nitriles, des hydrocarbures terpéniques, des composés hétérocycliques azotés ou souffrés, ainsi que des huiles essentielles d'origine naturelle. Le choix des ingrédients parfumants sera dicté par la nature de l'effet olfactif recherché, ainsi que par l'exigence de l'activité antimicrobienne globale de la base parfumante citée.

Selon le mode d'exécution de l'invention, la "BPAA" présentera une activité antimicrobienne d'au moins 60% telle que mesurée par la méthode du spray direct décrite ci-après. Il y a lieu cependant de noter que ces valeurs d'activité antimicrobienne sont données à titre indicatif, d'autres bases ayant une activité antimicrobienne inférieure pouvant se montrer parfaitement adaptées pour l'emploi dans les compositions parfumées de l'invention.

C'est ainsi que, d'une façon plus générale, la "BPAA" peut être définie comme étant une base parfumante qui contient une quantité prépondérante, de préférence 60% en poids ou plus, d'ingrédients parfumants connus pour avoir une action antimicrobienne. Ces derniers ne sauraient pas être énumérés ici de façon exhaustive. En effet, on trouve beaucoup d'exemples de tels ingrédients dans l'art antérieur (voir par exemple, EP 451 889 et les références y citées) et l'homme du métier est aujourd'hui à même de les choisir de façon à harmoniser l'effet olfactif qu'il recherche, lequel est également imparti par ladite base parfumante, et l'exigence d'action antimicrobienne de cette dernière, déterminée par la proportion de ces ingrédients par rapport à celle des ingrédients dont l'action est purement olfactive.

Par ailleurs, nous avons également établi, selon l'invention, que des ingrédients parfumants dont l'activité antimicrobienne était d'au moins 60%, telle que mesurée par la méthode du spray direct décrite plus loin, pouvaient également être employés avantageusement en tant qu'ingrédients parfumants à action antimicrobienne dans la "BPAA" mentionnée ci-dessus. Ainsi, l'invention a également pour objet une "BPAA", caractérisée en ce qu'elle contient au moins 60% en poids, par rapport au poids de la base, d'ingrédients parfumants dont l'activité antimicrobienne est d'au moins 60%, telle que mesurée par la méthode du spray direct et les compositions parfumées de l'invention qui contiennent cette "BPAA" sont des formes d'exécution préférées de l'invention.

Comme il a été cité précédemment, la "BPAA" remplit également la fonction d'impartir aux compositions parfumées selon l'invention les notes odorantes désirées. C'est ainsi qu'elle contiendra aussi des ingrédients parfumants d'usage courant dont l'effet est plutôt olfactif et qui, à l'instar des ingrédients parfumants microbicides susmentionnées, peuvent être choisis dans des classes chimiques déjà citées plus haut. Le choix de ces ingrédients sera dépendant de l'effet

olfactif recherché et de la nature du produit que l'on désire parfumer, ainsi que, bien entendu du goût du parfumeur créateur.

La "BPAA" présente dans les compositions parfumées selon l'invention peut être ajoutée à celles-ci soit telle quelle, soit sous forme microencapsulée ou encore sous forme d'émulsions telles que celles décrites dans le brevet européen no. 279 328 ou dans la demande de brevet européen no. 384 034. Les informations contenues dans ces documents ayant trait au contenu ou à la préparation desdites émulsions, ou à la microencapsulation de bases parfumantes à l'aide de la méthode y décrite, sont inclues ici par référence.

La "BPAA" sera ajoutée aux compositions parfumées selon l'invention dans les concentrations usuelles dans l'art. Les valeurs de ces concentrations dépendent de la nature du produit désodorisant ou antiperspirant final, ainsi que de l'effet olfactif recherché et l'homme du métier est à même de les choisir en fonction de ces paramètres. Des concentrations préférentielles ont déjà été citées plus haut.

Les compositions parfumées de l'invention peuvent également contenir des extraits de plantes riches en flavonoïdes. Il s'agit d'extraits de plantes solubles dans l'eau ou l'éthanol et qui ne contiennent plus d'huiles essentielles, lesquelles ont été préalablement enlevées par distillation à la vapeur. L'action antioxydante de ces extraits renforce le pouvoir désodorisant de la composition.

Des extraits de plantes aussi variées que le romarin, la sauge, la lavande et le lavandin, le thym, le thé vert, l'origan, la coriandre et le laurier peuvent être utilisés à cet effet. Des extraits des feuilles et fruits de l'olivier ou de l'eucalyptus, ainsi que des feuilles et des écorces des fruits de l'oranger, du citronnier et d'autres arbres d'agrumes, peuvent également être employés. Des résultats particulièrement avantageux ont été obtenus avec des extraits de romarin, dont on peut citer, à titre d'exemple, le produit commercial de FIS (Food Ingredients and Specialties S.A., Vevey, Suisse) connu sous le nom de Spice Extract AR.

Ces extraits de plantes seront ajoutés à la composition généralement sous forme de solutions dans l'éthanol ou l'eau, dans des concentrations de l'ordre de 0,05 à 0,5% en poids, par rapport au poids de la composition dans laquelle ils sont incorporés. Lorsque la base parfumante est utilisée sous forme microencapsulée, par exemple comme il est décrit dans EP 279 328 ou EP 384 034, l'extrait de plantes peut être incorporé dans l'émulsion encapsulante.

La composition parfumée selon l'invention peut encore contenir d'autres substances telles que des hydrocolloïdes. Il s'agit de substances polymériques naturelles ou synthétiques, telles que la carboxyméthylcellulose, les protéines hydrosolubles, certaines résines de plantes comme la gomme arabique, des alginates et carragénanes. L'utilisation de polyvinylpyrrolidone ou de ses dérivés quaternaires s'est aussi révelée avantageuse, renforçant l'effet antimicrobien de la composition.

Bien que la composition selon l'invention permette d'éviter l'utilisation d'agents bactéricides et antiperspirants conventionnels, il est clair que l'on peut, si désiré, lui ajouter de tels agents. Les concentrations dans lesquelles ces agents sont ajoutés seront alors typiquement inférieures aux concentrations usuelles, notamment en ce qui concerne les agents bactéricides et bactériostatiques.

La composition parfumée selon l'invention convient tout particulièrement à la préparation d'articles variés destinés aux soins corporels. Ces articles peuvent se présenter sous une multitude de formes différentes telles que savons, bâtonnets (sticks), roll-on (dispositifs à bille), aérosols ou vaporisateurs à pression mécanique ou manuelle. Suivant la nature de ces articles, la composition pourra être utilisée telle quelle, sous forme de solution alcoolique, éventuellement mélangée à un propulseur pour aérosol, ou encore mélangée à des ingrédients de nature variée couramment utilisés dans ces articles et illustrés dans les exemples présentés plus loin.

Ces articles désodorisants ou antiperspirants selon l'invention sont obtenus par mélange de leurs différents ingrédients au moyen d'appareillage conventionnel. La technique de mélange est en soi connue et toute explication détaillée est ici superflue. Des exemples détaillés sont présentés plus loin.

Par ailleurs, nous avons également découvert que la composition parfumée selon l'invention avait un emploi bien plus étendu et qu'elle pouvait notamment être utilisée avantageusement dans des détergents ou adoucissants textiles. Il a en effet été constaté que des textiles traités avec ces produits avaient une action désodorisante sensible lorsque portés sur la peau. Par ailleurs, il a été trouvé que la combinaison des phospholipides cationiques avec l'alcool cétylique ou myristique avait une action adoucissante sur les textiles et permettait ainsi de réduire, ou même de supprimer, l'usage de tensio-actifs quaternaires tels que le chlorure de stéaryl diméthyl ammonium, couramment employés pour cet effet adoucissant.

On a aussi constaté que ces deux ingrédients et, en particulier le phospholipide, exaltaient l'effet parfumant de la "BPAA", de sorte que l'effet odorant sur les tissus apparaissait beaucoup plus puissant et tenace, donc plus durable, que lorsque la "BPAA" était utilisée seule dans le parfumage du détergent ou de l'adoucissant textile.

La méthode du spray direct, ou "MSD", décrite par G. Holzner dans la référence citée auparavant, permet de déterminer l'efficacité bactéricide ou antimicrobienne de préparations cosmétiques appliquées sur la peau.

Grâce à une valve aérosol doseuse, la composition désodorisante ou antiperspirante prête à l'usage, ou une solution correspondante des matières actives, est giclée directement sur la surface du gel nutritif préalablement infecté avec le germe souhaité. Celui-ci est alors incubé à 37° C pendant 24 ou 48 heures en vue de l'évaluation subséquente de bactéries sur la surface circulaire traitée. L'action antimicrobienne de la composition parfumée est ensuite définie

par l'évaluation de la surface de la zone exempte de bactéries, par rapport à la surface totale circulaire traitée. Nous avons constaté que les compositions parfumées selon l'invention avaient une bonne activité désodorisante quand le rapport entre ces deux surfaces étaient d'environ 80% ou plus, lors du traitement du gel nutritif inoculé de germes avec ladite composition ou son principe actif.

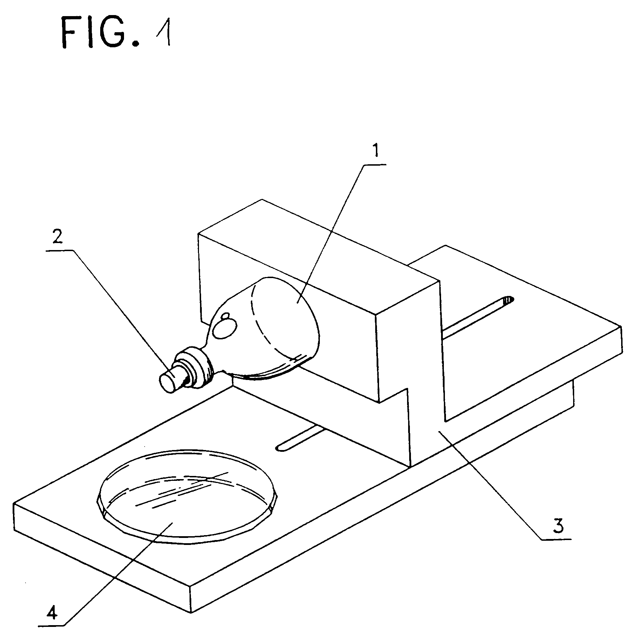

En référence à la figure, le flacon (1) aérosol en verre de 50 ml, muni d'une valve doseuse (2) avec diffuseur à orifice de sortie de 0,4 mm, est installé sur un dispositif de fixation (3) au-dessus d'une plaque de Petri (4), de manière que l'orifice de la valve se trouve à 9 cm au-dessus de la surface de la plaque. Dans ces conditions, il se forme un cône de spray qui marque, sur la plaque d'Agar, une superficie circulaire d'un diamètre d'environ 3 cm. La plaque de Petri est typiquement en matière plastique ou en verre, d'un diamètre de 9 cm.

On utilise une plaque de Petri conventionnelle, disponible dans le commerce et portant déjà le gel nutritif. A l'aide d'une pipette de Pasteur, on place dix gouttes de bouillon de culture (déjà inoculé avec le germe ou germes voulus et incubé pendant 48 h à 37°C) sur la plaque d'Agar et on les répartit uniformément sur celle-ci à l'aide d'une spatule en verre. On incube pendant 2h à 37°C et on place la plaque sur le dispositif de fixation (3).

Typiquement, pour mesurer l'activité antimicrobienne de la composition parfumée selon l'invention, on a procédé comme suit.

Lors de l'essai d'aérosols, on a placé la composition parfumée et le propulseur, par exemple dans les proportions respectives de 40 et 60%, dans le flacon (1). S'il s'agissait de compositions destinées à des bâtons ou sticks désodorisants ou antiperspirants, leurs matières actives, dans les proportions d'application respectives, ont été dissoutes dans l'éthanol à 95% et la solution disposée dans le flacon avec le propulseur comme décrit auparavant. La valve doseuse a alors été installée de façon connue en soi, en veillant à ce que l'extrémité du tube plongeur atteigne le fond du flacon. Ce dernier a été placé sur le dispositif de fixation en veillant à ce que l'orifice de la valve pointe dans la direction de la plaque de Petri. Cette dernière à été aspergée de solution active, en actionnant la valve une seule fois. La surface ainsi aspergée de la plaque a été mesurée et celle-ci ensuite incubée à 37° pendant 24 à 48 h. Après l'incubation, on a mesuré la surface de la zone de la plaque exempte de bactéries et on a définit ainsi l'action antimicrobienne de la composition parfumée selon l'invention.

A des fins de contrôle, on a toujours vaporisé sur des plaques de Petri, dans les mêmes conditions, une solution témoin composée, en général, d'éthanol à 95%.

Dans cette méthode on a utilisé des cultures des germes suivants :

Staphylococcus epidermidis
Staphylococcus aureus
Escherichia coli
Pseudomonas aeruginosa
Candida albicans
Corynebacterium pseudodiphteriae

Il s'agit d'un groupe incluant les organismes les plus couramment rencontrés sur la peau.

Comme cité précédemment, il est bien connu que, pour qu'une composition désodorisante ait une action efficace contre les mauvaises odeurs de la transpiration, elle doit avoir une activité bactéricide vis-à-vis d'un éventail de germes aussi large que possible et, en tout cas, englobant ceux cités ci-dessus. Nous avons maintenant établi que les compositions selon l'invention inhibent efficacement l'action de tous ces organismes.

Bien entendu, la méthode décrite ci-dessus peut également être employée pour déterminer l'activité antibactérienne des ingrédients individuels de la composition parfumée selon l'invention. C'est ainsi que nous avons pu établir que des compositions parfumées conformes à l'invention pouvaient être obtenues également lorsqu'elles contenaient, en plus du phospholipide cationique et de l'alcool gras, une base parfumante, cette base présentant une certaine activité antimicrobienne individuelle, telle que mesurée par la méthode du spray direct. Cette activité est alors mesurée dans les conditions décrites précédemment, en utilisant une solution de la "BPAA" dans l'éthanol, en tant que solution aspergeante. Bien entendu, la "BPAA" est présente dans cette solution dans les mêmes proportions pondérales que dans les compositions parfumées selon l'invention.

L'activité antimicrobienne des ingrédients parfumants destinés à être incorporés dans la base parfumante à action antimicrobienne, ou "BPAA", peut aussi être mesurée dans les conditions décrites auparavant, mais en utilisant, comme solution aspergeante, une solution de l'ingrédient parfumant dans l'éthanol, solution dans laquelle il est utilisé dans la même concentration que dans ladite "BPAA".

L'invention sera décrite plus en détail à l'aide des exemples présentés ci-après.

**Description sommaire du dessin**

La figure unique est une vue en perspective du dispositif employé dans la méthode du spray direct ("MSD").

## Manières de réaliser l'invention

Exemple 1

Base parfumante à action antimicrobienne ("BPAA")

On a préparé une base parfumante à action bactéricide par mélange des ingrédients suivants :

| Ingrédients | Parties en poids |
|---|---|
| Acétate de benzyle | 10,7 |
| Acétate de cyclohexyle* | 5,0 |
| Acétate de styrallyle* | 1,5 |
| Octanol* | 0,3 |
| Décanol* | 3,0 |
| Aldéhyde amylcinnamique | 4,0 |
| Ambrox® DL 1) à 10% dans le DIPG | 0,5 |
| Essence de bois de rose du Brésil* | 10,0 |
| Essence de camomille marocaine* | 2,5 |
| Caproate d'allyle* | 3,0 |
| Delphone 2)* | 5,0 |
| Absolue de feuilles d'oranger* | 1,5 |
| Géraniol* | 8,0 |
| Essence de girofle de Madagascar* | 5,0 |
| Essence de géranium bourbon* | 4,0 |
| Hedione® 3)* | 10,0 |
| Hydroxycitronellal | 2,5 |
| Iralia® 4) | 1,5 |
| Jasmin absolue synth. | 12,0 |
| Wardia® 5) | 6,0 |
| Essence d'ylang synth.* | 4,0 |
| Total | 100,0 |

* Ingrédients parfumants présentant une action antimicrobienne supérieure à 70% (lorsque dilués dans l'éthanol dans une concentration de 0,5%), telle que mesurée par la méthode du spray direct.
1) tétraméthyl perhydronaphtofurane ; origine : Firmenich SA, Genéve, Suisse.
2) pentyl cyclopentanone ; origine : Firmenich SA, Genéve, Suisse.
3) dihydrojasmonate de méthyle; origine : Firmenich SA, Genéve, Suisse.
4) méthylionone (mélange d'isoméres) ; origine : Firmenich SA, Genéve, Suisse.
5) origine : Firmenich SA, Genéve, Suisse.

Cette base parfumante, en solution dans l'éthanol à une concentration de 1,5%, a montré une activité antimicrobienne moyenne supérieure à 80%, telle que mesurée par la méthode du spray direct, comme il ressort du Tableau présenté ci-après dans lequel sont également indiquées les activités antimicrobienness des compositions selon les Exemples 2, 5 et 6.

TABLEAU I

| Composition | Staphylococcus epidermidis | Staphylococcus aureus | Escherichia coli | Pseudomonas aeruginosa | Candida albicans | Corynebacterium pseudodiphtariae |
|---|---|---|---|---|---|---|
| Base parfumante selon exemple 1 | ** | ** | + | * | ** | ** |
| Comp.1 selon exemple 2 | ** | ** | ** | * | ** | ** |
| Composition selon exemple 5 | ** | ** | ** | * | ** | ** |
| Composition selon exemple 6 | ** | ** | ** | * | ** | ** |

+ activité antimicrobienne supérieure à 70%, telle que mesurée par la MSD
* activité antimicrobienne supérieure à 80%, telle que mesurée par la MSD
** activité antimicrobienne supérieure à 90%, telle que mesurée par la MSD

Exemple 2

Composition parfumée pour spray à pression

Une composition parfumée à action désodorisante, destinée à être employée au moyen d'un appareil distributeur comportant un système gicleur mécanique ou à pression manuelle ("pumpspray" ou "squeeze bottle") a été préparée à l'aide des ingrédients suivants :

| Ingrédients | Composition 1 | Composition 2 |
|---|---|---|
| | (% en poids) | |
| Klucel EF 1) | -- | 0,3 |
| Eau | 22,0 | 20,9 |
| Luviquat Mono CP 2) | -- | 0,2 |
| Luviskol K-30 3) | -- | 0,4 |
| Gafquat HS-100 4) | -- | 0,2 |
| Ethanol 95% | 75,0 | 75,0 |
| Phospholipid EFA 5) | 0,4 | 0,4 |
| Extrait de romarin soluble dans l'alcool | 0,2 | 0,2 |
| Alcool myristique | 0,2 | 0,2 |
| Chremophor RH 40 6) | 0,7 | 0,7 |
| Parfum 7) | 1,3 | 1,3 |
| Tenox GT II 8) | 0,2 | 0,2 |
| Total | 100,0 | 100,0 |

1) hydroxypropylcellulose; origine : Hercules Co., USA
2) phosphate d'hydroxyéthyl cétyldimonium; origine : BASF AG, Allemagne
3) polyvinylpyrrolidone; origine : BASF AG, Allemagne
4) polyquaternium 28; origine : GAF Corp., USA
5) linoléamidopropyl PG-dimonium chlorure phosphate; origine : MONA Ind, USA.
6) huile de ricin hydrogéné et éthoxylé; origine : BASF AG, Allemagne
7) Evasion 111.092, de type agrumes, épicé, activité antimicrobienne supérieure à 70% ; origine : Firmenich SA, Genéve.
8) tocophérol; origine : Eastman Chemicals, USA

Les ingrédients susmentionnés ont été mélanges selon les méthodes courantes dans l'art et le mélange a été versé dans des récipients gicleurs. La composition 2 comprend le même principe actif que la composition 1, sous forme d'une émulsion encapsulante du type de celles décrites dans EP 384 034.

L'activité antimicrobienne moyenne de la composition 1, mesurée par la méthode du spray direct, était supérieure à 90% comme il ressort du Tableau 1.

Exemple 3

Composition parfumée pour roll-on

Une composition parfumée à action antiperspirante, destinée à être incorporée dans des distributeurs à bille de type roll-on, a été préparée avec les ingrédients suivants:

| Ingrédients | Parties en poids |
|---|---|
| Micro Dry Ultrafine 1) | 20,0 |
| Dow Corning 345 2) | 73,3 |
| Ethanol anhydre | 1,5 |
| Bentone 38 3) | 2,7 |
| Parfum 4) | 0,5 |
| Base active | 2,0 |
| Total | 100 |

1) chlorhydrate d'aluminium; origine : Reheis Chem. Co., USA
2) cyclométhicone; origine : Dow Chemicals, USA
3) quaternium 18-hectorite; origine : Rheox Inc., USA
4) Lixia liquide, type aldéhydé, floral; activité antimicrobienne supérieure à 70%; origine : Firmenich SA, Genéve

Les ingrédients susmentionnés ont été mélangés selon les techniques courantes, la base active étant préparée à priori comme il est décrit ci-après. Le mélange a ensuite été versé dans des récipients distributeurs roll-on.

Base active

Cette base a été obtenue comme il est décrit ci-après, à l'aide des ingrédients suivants:

| Ingrédients | Poids |
|---|---|
| Eau | 115,000 |
| Tylose H-10 1) | 1,000 |
| Extrait de romarin soluble dans l'eau | 25,000 |
| Sucre | 1,000 |
| Capsul 2) | 49,175 |
| Hostaphat KL-340 3) | 0,200 |
| Phospholipid EFA 4) | 15,000 |
| Acide citrique | 1,000 |
| Aloe 10 X 5) | 10,000 |
| Allantoin micronisé 6) | 5,000 |
| Antimousse 7) | 0,100 |
| Parfum 8) | 25,000 |
| BNT 9) | 0,025 |
| Tenox GT II 10) | 2,500 |
| Total | 250,000 |

1) hydroxyéthylcellulose; origine : Höchst AG, Allemagne
2) Na-octenylsuccinate d'amidon; origine : National Starch Inc., USA
3) trilaureth-4-phosphate; Höchst AG, Allemagne
4) voir exemple 2
5) extrait d'aloe; origine : Terry Corp., USA
6) origine : Chemie Linz, Autriche
7) émulsion d'huile de silicone; origine : Bayer AG, Allemagne
8) voir plus haut
9) p-tert-butylhydroxytoluéne; origine : Shell Co., Hollande
10) tocophérol; origine : Eastman Chemicals, USA

Les ingrédients de cette base active ont été dissous l'un après l'autre dans l'ordre indiqué. L'émulsion résultante a été bien homogenéisée à l'aide d'un mixer de type Silverson, à température ambiante.

L'émulsion a ensuite été chauffée à 60°C et atomisée dans un atomiseur conventionnel de type Büchi, la température de l'air d'entrée étant de 220°C et la température de l'air de sortie, mélangé à la poudre, étant de 120°C. On a ainsi obtenu une poudre contenant tous les ingrédients susmentionnés, à l'exception de l'eau. Cette poudre était constituée de microcapsules hydrosolubles contenant le parfum.
La poudre ainsi obtenue a ensuite été incorporée à la composition parfumée, dans la proportion indiquée.

Exemple 4

Composition parfumée pour spray aérosol

Une composition parfumée à action antiperspirante, destinée à être incorporée dans un distributeur de type "spray" a été préparée à l'aide des ingrédients suivants:

| Ingrédients | Parties en poids |
|---|---|
| Micro Dry Ultrafine [1)] | 8,0 |
| Dow Corning 345 [2)] | 19,6 |
| Parfum [3)] | 0,4 |
| Base active [4)] | 2,0 |
| Propulseur propane/butane [5)] | 70,0 |
| Total | 100,0 |

1), 2), 3) et 4) voir exemple 3
5) mélange 2,5 bar

Le mélange, préparé selon les méthodes usuelles, a été versé dans des récipients aérosol.

Exemple 5

Composition parfumée pour spray aérosol

Une composition parfumée à action désodorisante, destinée à être appliquée à l'aide d'un distributeur du type spray, a été préparée par mélange des ingrédients suivants :

| Ingrédients | Parties en poids |
|---|---|
| Klucel EF [1)] | 0,30 |
| Rexene Fe 3 spec. [2)] | 0,03 |
| Eau déminéralisée | 5,00 |
| Phospholipid PTC [3)] | 0,30 |
| Luviskol K 30 [4)] | 0,50 |
| Gafquat HS-100 [5)] | 0,40 |
| Ethanol 95% | 90,17 |
| D,L-$\alpha$-Tocophérol [6)] | 0,20 |
| Alcool cétylique | 1,00 |
| Extrait de romarin soluble dans l'alcool [7)] | 0,10 |
| Parfum [8)] | 2,00 |
| Total | 100,00 |

1) voir exemple 2
2) dihydroxyéthyl glycine; origine : Rexolin
3) cocamidopropyl PG-dimonium chlorure phosphate; origine : Mona Ind., USA
4) voir exemple 2
5) voir exemple 2
6) origine : Hoffmann-La Roche, Suisse
7) Spice extract AR; origine : FIS, Vevey Suisse
8) base parfumante décrite à l'exemple 1

Les ingrédients susmentionnés ont été mélangés de façon à obtenir un mélange parfaitement homogène. Ce mélange a été versé dans un distributeur aérosol avec adjonction de diméthyléther, dans les proportions respectives de 60:40.

L'activité antimicrobienne moyenne de cette composition était supérieure à 90%, telle que mesurée par la méthode du spray direct (voir Tableau 1).

Exemple 6

Composition parfumée pour spray aérosol

Une composition parfumée à action désodorisante, destinée à être appliquée à l'aide d'un distributeur de type spray, a été préparée avec les ingrédients suivants:

| Ingrédients | Parties en poids |
|---|---|
| Ethanol à 95% | 96,6 |
| Phospholipid PTC 1) | 0,2 |
| Extrait de romarin soluble dans l'alcool 2) | 0,1 |
| D, L-α-Tocophérol 3) | 0,2 |
| Alcool myristique | 1,0 |
| Parfum 4) | 1,9 |
| Total | 100,0 |

1, 2), 3) et 4) voir exemple 5

Le mélange de ces ingrédients a été versé dans un distributeur avec le propulseur constitué par un mélange isobutane/propane 90:10. Les proportions relatives de mélange actif et propulseur étaient de 65% et 35% respectivement. La composition parfumée ainsi obtenue présentait une activité antimicrobienne moyenne supérieure à 90% contre tous les organismes cités auparavant.

Exemple 7

Composition parfumée pour savon

On a préparé un savon parfumé avec les ingrédients suivants :

| | Ingrédients | Parties en poids |
|---|---|---|
| I | Base de salon 1) | 93,0 |
| II | Parfum 2) | 1,5 |
| | Alcool cétylique | 0,5 |
| | Phospholipid PTC ou EFA 3) | 5,0 |
| | Total | 100,0 |

1) Prisavon 9250 ; origine : Unichema
2) base parfumante selon l'Exemple 1
3) voir exemples précédents

On a mélangé et chauffé légèrement la partie II pour obtenir une émulsion homogène. Les parties I et II ont alors été mélangées à l'aide d'un laminoir à 3 rouleaux. Finalement, la pâte ainsi obtenue a été extrudée dans une boudineuse et le savon frappé.
Une solution dans l'éthanol de la base active de ce savon (partie II), dans laquelle les trois ingrédients ont été utilisés dans les proportions indiquées, présentait une activité antimicrobienne proche de 100%.
D'autre part, un test de type "Hill Top" exécuté sur un panel de 5 personnes, a montré que le savon ainsi préparé présentait une valeur désodorisante moyenne de 2,8.

Exemple 8

Composition parfumée pour un adoucissant textile liquide

Une composition parfumée destinée a être utilisée dans un adoucissant textile liquide a été préparée à l'aide des ingrédients suivants :

| | Ingrédients | Parties en poids |
|---|---|---|
| I | Parfum 1) | 2,0 |
| II | Phospholipid PTC 2) | 15,0 |
| | Alcool cétylique ou myristique | 5,0 |
| III | Eau déminéralisée | 77,8 |
| | Acide citrique | 0,2 |
| | Total | 100 |

1) base parfumante selon l'exemple 1
2) voir exemple précédent

On a chauffé les parties II et III séparément à 60°C et versé la partie III dans la II. On a agité jusqu'à ce que la température ait atteint 40°C et ajouté la partie I, en maintenant l'agitation jusqu'à obtenir un mélange homogène et à ce que la température soit descendue à 20°C environ.
Pour application, 30 à 50 g de cette émulsion ont été ajoutés à l'eau du dernier rinçage dans un cycle de lavage de linge dans une machine à laver. Alternativement, la composition parfumée préparée comme décrit ci-dessus peut être ajoutée à une base adoucissante liquide d'usage courant.
Le linge traité avec la composition parfumée décrite ci-dessus avait une action désodorisante sensible lorsque porté sur la peau.
Par ailleurs, et en parallèle, un autre lot de tissus identique a été traité de la même façon avec un adoucissant textile contenant la même quantité d'une émulsion où la partie II ci-dessus avait été remplacée par un tensioactif quaternaire classique, à savoir le Praepagen WK de Höchst (chlorure de stéaryldiméthylammonium).
Les deux lots de tissus ont été évalués à l'aveugle par un panel d'experts parfumeurs, lequel a constaté que l'odeur des tissus traités avec la composition selon la présente invention était remarquablement plus puissante que celle des tissus traités avec la composition contenant les ingrédients classiques, et que cette odeur était beaucoup plus durable sur les tissus secs.

Exemple 9

Composition parfumée pour adoucissant textile

Une composition destinée à un adoucissant textile pour usage dans un séchoir à linge a été préparée avec les ingrédients suivants :

| Ingrédients | Parties en poids |
|---|---|
| Parfum 1) | 15,0 |
| Phospholipid PTC 2) | 40,0 |
| Alcool cétylique | 34,8 |
| Acide stéarique | 20,0 |
| Acide citrique | 0,2 |
| Total | 100,0 |

1) et 2) voir exemple 7

On a mélangé les ingrédients et chauffé légèrement (au-dessous de 70°C) pour obtenir un mélange homogène et liquide à cette température. Des tissus du type "non-woven" de 25 cm x 25 cm ont été ensuite imprégnés avec 1,5 à 2g de ce mélange. Les tissus ont été ajoutés à du linge humide dans le tambour d'un séchoir électrique. Le linge séché en présence de ces tissus était doux et a montré une action désodorisante sensible lorsque porté sur le corps.

Exemple 10

Afin de tester le pouvoir désodorisant de la composition parfumée selon l'invention, un panel de vingt individus a appliqué sous une aisselle, à l'aide d'un spray aérosol, la composition 1 décrite dans l'exemple 2, l'autre aisselle n'ayant pas été traitée ou ayant été traitée avec un produit témoin contenant le même parfum, mais faisant usage d'ingrédients désodorisants conventionnels. Chaque individu devait ensuite comparer, au terme de 8, 12 et 24 heures, l'odeur des deux aisselles et indiquer laquelle présentait l'odeur la plus agréable. Au bout d'une semaine, le même test a été répété, mais en intervertissant les sprays, ceci dans le but d'éliminer les effets éventuels résultant du fait que, chez une même personne, les deux aisselles peuvent présenter des odeurs différentes.
A la fin des deux semaines de tests on a pu conclure qu'environ 70% des membres du panel préféraient l'odeur de l'aisselle traitée avec le produit selon l'invention, 25% avaient trouvé une odeur identique pour les deux aisselles et les restants avaient préféré l'odeur de l'aisselle traitée avec le produit témoin.

**Revendications**

1. Composition parfumée caractérisée en ce qu'elle contient un phospholipide cationique, une base parfumante à action antimicrobienne ("BPAA") et un alcool gras ayant de 10 à 22 atomes de carbone, la "BPAA" ayant une activité antimicrobienne d'au moins 60% telle que mesurée par la méthode du spray direct ("MSD").

2. Composition selon la revendication 1, caractérisée par une activité antimicrobienne d'au moins 80%, telle que mesurée par la méthode du spray direct.

3. Composition selon la revendication 1 ou 2, caractérisée en ce que le phospholipide cationique est un composé de formule

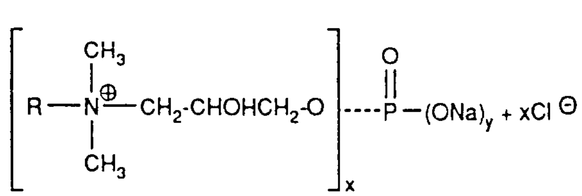

(I)

dans laquelle R est un radical linoléamidopropyle ou cocamidopropyle et $x + y = 3$ .

4. Composition selon l'une des revendications précédentes, caractérisée en ce que l'alcool gras est l'alcool myristique ou l'alcool cétylique.

5. Composition selon l'une des revendications 1 à 4, caractérisée en ce qu'elle contient de 0,05 à 5% en poids de phospholipide, par rapport au poids de la composition.

6. Composition selon l'une des revendications 1 à 4, caractérisée en ce qu'elle contient de 0,1 à 5% en poids de "BPAA", par rapport au poids de la composition.

7. Composition selon l'une des revendications 1 à 4, caractérisée en ce qu'elle contient de 0,02 à 2% en poids d'alcool gras, par rapport au poids de composition.

8. Composition selon la revendication 1, caractérisée en ce qu'elle contient environ 0,05 à 2% en poids de phospholipide, environ 0,5 à 5% en poids de "BPAA" et environ 0,05 à 2% en poids d'alcool gras, par rapport au poids de composition, et présente une activité antimicrobienne d'au moins 90%, telle que mesurée par la méthode du spray direct.

9. Composition selon la revendication 1, caractérisée en ce que la "BPAA" contient au moins 60% en poids, par rapport au poids de "BPAA", d'ingrédients parfumants ayant chacun une activité antimicrobienne d'au moins 60% telle que mesurée par la méthode du spray direct.

10. Composition selon l'une des revendications précédentes, caractérisée en ce qu'elle contient un extrait de plantes riche en flavonoïdes.

11. Composition selon la revendication 10, caractérisée en ce que l'extrait de plantes est un extrait de romarin.

12. Article désodorisant ou antiperspirant destiné aux soins corporels contenant une composition parfumée selon l'une des revendications 1 à 11.

13. Article selon la revendication 12, sous forme d'un savon, d'un batonnet (stick), d'un roll-on, d'un aérosol ou d'un vaporisateur à pression mécanique ou manuelle.

14. Détergent ou adoucissant textile contenant une composition parfumée selon l'une des revendications 1 à 11.

15. Base parfumante à action antimicrobienne ("BPAA"), caractérisée en ce qu'elle contient au moins 60% en poids, par rapport au poids de la "BPAA", d'ingrédients parfumants dont l'activité antimicrobienne est d'au moins 60% telle que mesurée par la méthode du spray direct.

## Claims

1. Perfumed composition characterized in that it contains a cationic phospholipid, a perfuming base with antimicrobial action ("PBAA") and a fatty alcohol having from 10 to 22 carbon atoms, the "PBAA" having an antimicrobial activity of at least 60%, as measured by the direct spray method ("DSM").

2. Composition according to claim 1, characterized by an antimicrobial activity of at least 80%, as measured by the direct spray method.

3. Composition according to claim 1 or 2, characterized in that the cationic phospholipid is a compound of formula

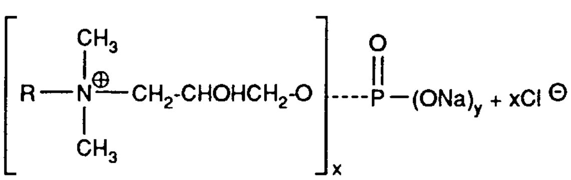

(I)

wherein R is a linoleamidopropyl or cocamidopropyl radical and $x + y = 3$ .

4. Composition according to any one of the preceding claims, characterized in that the fatty alcohol is myristyl or cetyl alcohol.

5. Composition according to any one of claims 1 to 4, characterized in that it contains from 0.05 to 5% by weight of phospholipid, relative to the weight of the composition.

6. Composition according to any one of claims 1 to 4, characterized in that it contains from 0.1 to 5% by weight of "PBAA", relative to the weight of the composition.

7. Composition according to any one of claims 1 to 4, characterized in that it contains from 0.02 to 2% by weight of fatty alcohol, relative to the weight of the composition.

8. Composition according to claim 1, characterized in that it contains about 0.05 to 2% by weight of phospholipid, about 0.5 to 5% by weight of "PBAA" and about 0.05 to 2% by weight of fatty alcohol, relative to the weight of the composition, and presents an antimicrobial activity of at least 90%, as measured by the direct spray method.

9. Composition according to claim 1, characterized in that the "PBAA" contains at least 60% by weight, relative to the weight of "PBAA", of perfuming ingredients having each an antimicrobial activity of at least 60% as measured by the direct spray method.

10. Composition according to any one of the preceding claims, characterized in that it contains a plant extract rich in flavonoids.

11. Composition according to claim 10, characterized in that the plant extract is a rosemary extract.

12. Deodorant or antiperspirant article intended for body care, containing a perfumed composition according to any one of claims 1 to 11.

13. Article according to claim 12, in the form of a soap, a stick, a roll-on, an aerosol or a mechanical or manual pressure vaporizer.

14. Detergent or fabric softener containing a perfumed composition according to any one of claims 1 to 11.

15. Perfuming base with antimicrobial action ("PBAA"), characterized in that it contains at least 60% by weight, relative to the weight of the "PBAA", of perfuming ingredients having an antimicrobial activity of at least 60% as measured by the direct spray method.

## Patentansprüche

1. Parfümierte Zusammensetzung, dadurch gekennzeichnet, dass diese ein kationisches Phospholipid, eine Riechstoffbasis mit antimikrobieller Wirkung ("BPAA") und einen Fettalkohol mit 10 bis 22 Kohlenstoffatomen enthält, wobei die "BPAA" weist eine antimikrobielle Aktivität, gemessen mittels Direktsprühmethode ("DSM"), von mindestens 60% aufweist.

2. Zusammensetzung gemäss Patentanspruch 1, gekennzeichnet durch eine antimikrobielle Aktivität, gemessen mittels Direktsprühmethode, von mindestens 80%.

3. Zusammensetzung gemäss Patentanspruch 1 oder 2, dadurch gekennzeichnet, dass das kationische Phospholipid eine Verbindung der Formel

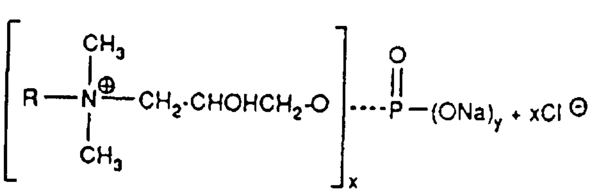

(I)

ist, worin R für einen Linolamidopropyl- oder einen Cocamidopropylrest steht und x + y = 3 ist.

4. Zusammensetzung gemäss einem der vorhergehenden Patentansprüche, dadurch gekennzeichnet, dass der Fettalkohol Myristinalkohol oder Cetylalkohol ist.

5. Zusammensetzung gemäss einem der Patentansprüche 1 bis 4, dadurch gekennzeichnet, dass diese, bezogen auf das Gewicht der Zusammensetzung, 0,05 bis 5 Gew.% Phospholipid enthält.

6. Zusammensetzung gemäss einem der Patentansprüche 1 bis 4, dadurch gekennzeichnet, dass diese, bezogen auf das Gewicht der Zusammensetzung, 0,1 bis 5 Gew.% "BPAA" enthält.

7. Zusammensetzung gemäss einem der Patentansprüche 1 bis 4, dadurch gekennzeichnet, dass diese, bezogen auf das Gewicht der Zusammensetzung, 0,02 bis 2 Gew.% Fettalkohol enthält.

8. Zusammensetzung gemäss Patentanspruch 1, dadurch gekennzeichnet, dass diese, bezogen auf das Gewicht der Zusammensetzung, ca. 0,05 bis 2 Gew.% Phospholipid, ca. 0,5 bis 5 Gew.% "BPAA" und ca. 0,05 bis 2 Gew.% Fettalkohol enthält und eine antimikrobielle Aktivität, gemessen mittels Direktsprühmethode, von mindestens 90% aufweist.

9. Zusammensetzung gemäss Patentanspruch 1, dadurch gekennzeichnet, dass die "BPAA", bezogen auf das Gewicht von "BPAA", mindestens 60 Gew.% Riechstoffbestandteile enthält, wobei jeder eine antimikrobielle Aktivität, gemessen mittels Direktsprühmethode, von mindestens 60% aufweist.

10. Zusammensetzung gemäss einem der vorhergehenden Patentansprüche, dadurch gekennzeichnet, dass diese einen Extrakt von Pflanzen, welche reich sind an Flavonoiden, enthält.

11. Zusammensetzung gemäss Patentanspruch 10, dadurch gekennzeichnet, dass der Pflanzenextrakt ein Rosmarinextrakt ist.

12. Ein desodorierender und die Transpiration unterdrükkender Artikel, bestimmt für die Körperpflege, enthaltend eine parfümierte Zusammensetzung gemäss einem der Patentansprüche 1 bis 11.

13. Ein Artikel gemäss Patentanspruch 12, in Form einer Seife, eines Sticks, eines Roll-on, eines Aerosols oder eines Zerstäubers mit mechanischer oder manueller Druckbetätigung.

14. Reinigungsmittel oder Textilauffrischungsmittel, enthaltend eine parfümierte Zusammensetzung gemäss einem der Patentansprüche 1 bis 11.

15. Riechstoffbasis mit antimikrobieller Wirkung ("BPAA"), dadurch gekennzeichnet, dass diese, bezogen auf das Gewicht von "BPAA", mindestens 60 Gew.% Riechstoffbestandteile enthält, deren antimikrobielle Aktivität, gemessen mittels Direktsprühmethode, mindestens 60% beträgt.

FIG. 1